# EUROPEAN PATENT APPLICATION

(11) **EP 4 772 202 A1**
(43) Date of publication of application: **08.07.2026**
(21) Application number: 23950473.1
(22) Date of filing: 23.11.2023
(51) Int. Cl.: A61L 27/20, A61L 31/10

(54) **USE OF KONJAC GLUCOMANNAN AND DERIVATIVE THEREOF, GLUCOMANNAN CAPRYLATE, NANOPARTICLES AND COATING OBTAINED FROM GLUCOMANNAN CAPRYLATE, PREPARATION METHOD FOR NANOPARTICLES, AND USE OF GLUCOMANNAN CAPRYLATE, NANOPARTICLES OR COATING**

(30) Priority: 29.08.2023 CN 202311098026
(71) Applicant: University of Macau, Taipa 999078 (MO)
(72) Inventor: WANG, Chunming, Taipa 999078 (MO); LIU, Yu, Taipa 999078 (MO); ZHANG, Zhe, Taipa 999078 (MO)
(74) Representative: Schlich
(86) International application number: PCT/CN2023/133704
(87) International publication number: WO 2025/043903

(57) **Abstract**

A use of konjac glucomannan and a derivative thereof, glucomannan caprylate, nanoparticles and a coating obtained from the glucomannan caprylate, a preparation method for the nanoparticles, and a use of the glucomannan caprylate, the nanoparticles or the coating, relating to the technical field of medicine and material chemistry. The konjac glucomannan and the derivative thereof can be used as extracellular matrix fillers, and glucomannan caprylate obtained by modifying the konjac glucomannan by means of an esterification reaction, and nanoparticles or a glucomannan caprylate coating further obtained from the glucomannan caprylate can both be used for preparing drugs or materials for regulating functions of nucleus pulposus cells, and in particular, can be used for preparing drugs or materials for relieving or treating intervertebral disc degeneration. The glucomannan caprylate has the characteristics of being non-toxic, easily available and highly histocompatible, and can be prepared into different dosage forms according to requirements. The present disclosure provides a novel idea to improve the microenvironment of intervertebral disc degeneration, and also provides a novel use of a biomaterial based on a polysaccharide derivative.

## Description

### Cross Reference to Related Applications

The present application claims priority to Chinese Patent Application No. 202311098026 X, filed with the China National Intellectual Property Administration on August 29, 2023 and entitled "USE OF KONJAC GLUCOMANNAN AND DERIVATIVE THEREOF, GLUCOMANNAN CAPRYLATE, NANOPARTICLES AND COATING OBTAINED FROM GLUCOMANNAN CAPRYLATE, PREPARATION METHOD FOR NANOPARTICLES, AND USE OF GLUCOMANNAN CAPRYLATE, NANOPARTICLES OR COATING", which is incorporated herein by reference in its entirety.

### Technical Field

The present disclosure relates to the technical field of medicine and material chemistry, in particular to a use of konjac glucomannan and a derivative thereof, glucomannan caprylate, nanoparticles and a coating obtained from the glucomannan caprylate, a preparation method for the nanoparticles, and a use of the glucomannan caprylate, the nanoparticles or the coating.

### Background Art

Lumbar intervertebral disc degeneration is one of the most important causes of low back pain, and epidemiological surveys show that approximately 80% of adults have a history of more than one occurrence of low back pain. As a disorder of the locomotor system closely associated with aging and exercise, with the rapid development of an aging population and the popularisation of nationwide fitness programs, an increasing number of individuals will face the problem of intervertebral disc degeneration and corresponding nerve compression symptoms. A typical feature of intervertebral disc degeneration is the progressive degeneration of intervertebral disc tissues, particularly nucleus pulposus tissues. Currently, medication and physical therapy can only alleviate symptoms in some patients but cannot effectively intervene in the process of tissue degeneration, and most patients have no choice but to undergo surgical treatment as a final treatment option. However, local mechanical structural instability caused by postoperative tissue loss, continuous degeneration of remaining degenerated nucleus pulposus tissues, and postoperative microenvironmental disturbance are important reasons for aggravating tissue degeneration, causing re-herniation, and leading to reoperation. How to effectively regulate the postoperative degenerative microenvironment, prevent recurrence, and promote the repair of nucleus pulposus tissue is currently the mainstream strategy for enhanced recovery.

Pathological studies have found that during intervertebral disc degeneration, the extracellular matrix (ECM) of the nucleus pulposus tissue undergoes accelerated degradation, the number of nucleus pulposus cells decreases, and the phenotype begins to be lost. Therefore, typical changes including decreased MR T2 signal intensity of the nucleus pulposus and reduced intervertebral disc space height can be clinically observed. Basic research further reveals: as the degeneration progresses, matrix degradation-related enzymes (e.g., MMP2, HYAL2) within the nucleus pulposus tissue are gradually highly expressed, while the content of ECM components such as collagen and hyaluronic acid gradually decreases. In view of the changing characteristics of ECM, researchers have proposed the use of injectable, filling materials to fill the postoperative defect of intervertebral disc to achieve the purposes of maintaining height, improving the degenerative microenvironment, and improving treatment efficiency. However, to date, no effective related materials have been reported.

In view of this, the present disclosure is hereby provided.

### Summary

The present disclosure provides the use of a konjac glucomannan and a derivative thereof as extracellular matrix fillers.

Optionally, the derivative of konjac glucomannan is a modified konjac glucomannan.

Optionally, the modification is to modify a polysaccharide molecule of the konjac glucomannan to introduce an additional functional group; optionally, the functional group is an acetyl group or an octanoyl group.

Optionally, the derivative of konjac glucomannan is a glucomannan caprylate.

Optionally, the degree of substitution of the glucomannan caprylate is 1.0-2.5; optionally, the degree of substitution of the glucomannan caprylate is 1.5-2.5; optionally, the degree of substitution of the glucomannan caprylate is 1.6-1.8.

Optionally, the konjac glucomannan and the derivative thereof are in the form of nanoparticles or a coating.

The present disclosure provides a glucomannan caprylate, which is obtained by modifying a konjac glucomannan by means of an esterification reaction.

Optionally, the degree of substitution of the glucomannan caprylate is 1.0-2.5; optionally, the degree of substitution of the glucomannan caprylate is 1.5-2.5; optionally, the degree of substitution of the glucomannan caprylate is 1.6-1.8.

The present disclosure provides a method for synthesising the glucomannan caprylate according to above embodiment, comprising the step of: modifying a konjac glucomannan with an octanoyl group.

Optionally, the konjac glucomannan is reacted with trifluoroacetic anhydride and octanoic acid, the reaction solution obtained from the reaction is subjected to solid-liquid separation, the liquid phase is collected for first alcohol precipitation, a first precipitate obtained from the first alcohol precipitation is dissolved in chloroform to remove a product insoluble in chloroform, subsequently the liquid phase dissolved in chloroform is subjected to second alcohol precipitation, and a second precipitate obtained is collected.

Optionally, the konjac glucomannan is added to a first reaction solution obtained after a reaction of trifluoroacetic anhydride with octanoic acid for further reaction to obtain a second reaction solution; the second reaction solution is subjected to solid-liquid separation, the liquid phase is collected for first alcohol precipitation, a first precipitate obtained from the first alcohol precipitation is dissolved in chloroform to remove a product insoluble in chloroform, subsequently the liquid phase dissolved in chloroform is subjected to second alcohol precipitation, and a second precipitate obtained is collected.

Optionally, the reaction of the trifluoroacetic anhydride with the octanoic acid is carried out at 50°C-55°C for 15-25 min.

Optionally, the reaction of the konjac glucomannan with the first reaction solution is carried out at 50°C-55°C for 3-4 h.

Optionally, the amount ratio of the konjac glucomannan, the trifluoroacetic anhydride, and the octanoic acid is 500 mg : (8-12) mL : (12-14) mL.

The present disclosure provides a nanoparticle, which is obtained by subjecting the glucomannan caprylate according to the above embodiment to a nanonisation treatment.

The present disclosure provides a method for preparing the nanoparticle according to the above embodiment, comprising the step of: subjecting the glucomannan caprylate to a nanonisation treatment.

Optionally, the glucomannan caprylate is subjected to an ultrasonic nanonisation treatment in the presence of polyvinyl alcohol, an organic solvent is removed, and drying is carried out.

The present disclosure provides a glucomannan caprylate coating, which is obtained by coating with the glucomannan caprylate according to the above embodiment.

The present disclosure provides the use of the glucomannan caprylate according to the above embodiment or the nanoparticle according to the above embodiment or the glucomannan caprylate coating according to the above embodiment in the preparation of a drug or material for regulating functions of nucleus pulposus cells.

Optionally, the glucomannan caprylate or the nanoparticle or the glucomannan caprylate coating is used for preparing a drug or material for regulating the adhesion and physiological functions of nucleus pulposus cells.

Optionally, the glucomannan caprylate or the nanoparticle or the glucomannan caprylate coating is used for preparing a drug or material for protecting the phenotype of nucleus pulposus cells by interfering with cell-matrix and cell-cell interactions.

Optionally, the glucomannan caprylate or the nanoparticle or the glucomannan caprylate coating is used for preparing a drug or material for highly expressing a nucleus pulposus phenotype; optionally, the nucleus pulposus phenotype comprises Collagen2.

Optionally, the glucomannan caprylate or the nanoparticle or the glucomannan caprylate coating is used for preparing a drug or material for enriching milk fat globule epidermal growth factor 8 secreted by nucleus pulposus cells; wherein the nucleus pulposus phenotype comprises Collagen2.

In an optional embodiment, the material is a repair material.

The present disclosure provides the use of the glucomannan caprylate according to the above embodiment or the nanoparticle according to the above embodiment or the glucomannan caprylate coating according to the above embodiment in the preparation of a drug or material for relieving or treating intervertebral disc degeneration.

Optionally, the glucomannan caprylate or the nanoparticle or the glucomannan caprylate coating is used for preparing a drug for delaying water loss of a nucleus pulposus tissue, or, for preparing a drug for alleviating a decrease in intervertebral disc space height.

Optionally, the material is a repair material.

The present disclosure provides a drug, wherein an active ingredient of the drug comprises the glucomannan caprylate according to the above embodiment or comprises the glucomannan caprylate nanoparticle according to the above embodiment or the glucomannan caprylate coating according to the above embodiment.

The present disclosure provides a repair material, wherein an active component of the repair material comprises the glucomannan caprylate according to the above embodiment or comprises the nanoparticle according to the above embodiment or the glucomannan caprylate coating according to the above embodiment.

The present disclosure provides a method for regulating functions of nucleus pulposus cells, comprising administering to a subject in need thereof or applying to a subject in need thereof a therapeutically effective amount of the glucomannan caprylate according to the above embodiment or the nanoparticle according to the above embodiment or the glucomannan caprylate coating according to the above embodiment.

Optionally, the administering or applying is carried out in the form of a drug or material, respectively.

Optionally, the regulating functions of nucleus pulposus cells is selected from one or more of:
regulating the adhesion and physiological functions of nucleus pulposus cells;
protecting the phenotype of nucleus pulposus cells by interfering with cell-matrix and cell-cell interactions;
highly expressing a nucleus pulposus phenotype; optionally, the nucleus pulposus phenotype comprises Collagen2;
enriching milk fat globule epidermal growth factor 8 secreted by nucleus pulposus cells; wherein the nucleus pulposus phenotype comprises Collagen2.

Optionally, the material is a repair material.

The present disclosure provides a method for relieving or treating intervertebral disc degeneration, comprising administering to a subject in need thereof or applying to a subject in need thereof a therapeutically effective amount of the glucomannan caprylate according to the above embodiment or the nanoparticle according to the above embodiment or the glucomannan caprylate coating according to the above embodiment.

Optionally, the administering or applying is carried out in the form of a drug or material, respectively.

Optionally, the relieving or treating intervertebral disc degeneration is selected from delaying water of a nucleus pulposus tissue and/or alleviating a decrease in intervertebral disc space height.

Optionally, the material is a repair material.

The present disclosure provides the glucomannan caprylate according to the above embodiment or the nanoparticle according to above embodiment or the glucomannan caprylate coating according to above embodiment, for use in regulating functions of nucleus pulposus cells.

Optionally, the administering or applying is carried out in the form of a drug or material, respectively.

Optionally, the regulating functions of nucleus pulposus cells is selected from one or more of:
regulating the adhesion and physiological functions of nucleus pulposus cells;
protecting the phenotype of nucleus pulposus cells by interfering with cell-matrix and cell-cell interactions;
highly expressing a nucleus pulposus phenotype; optionally, the nucleus pulposus phenotype comprises Collagen2;
enriching milk fat globule epidermal growth factor 8 secreted by nucleus pulposus cells; wherein the nucleus pulposus phenotype comprises Collagen2.

Optionally, the material is a repair material.

The present disclosure provides the glucomannan caprylate according to the above embodiment or the nanoparticle according to the above embodiment or the glucomannan caprylate coating according to the above embodiment, for use in relieving or treating intervertebral disc degeneration.

Optionally, the administering or applying is carried out in the form of a drug or material, respectively.

Optionally, the relieving or treating intervertebral disc degeneration is selected from delaying water of a nucleus pulposus tissue and/or alleviating a decrease in intervertebral disc space height.

Optionally, the material is a repair material.

### Brief Description of the Drawings

In order to more clearly illustrate the technical solutions of the examples of the present disclosure, the drawings used in the examples will be briefly introduced below. It should be understood that the following drawings only show certain examples of the present disclosure, and therefore should not be considered as limiting the scope. For a person skilled in the art, other related drawings can also be obtained according to the drawings without creative efforts.
FIG. 1 shows a mass spectrum of the enzymatic hydrolysate of hyaluronic acid in Example 1;
FIG. 2 shows a mass spectrum of the enzymatic hydrolysate of KGM in Example 1;
FIG. 3 shows a comparison of the infrared spectra of KGM and GMOC in Example 2;
FIG. 4 shows a comparison of the nuclear magnetic resonance spectra of KGM and GMOC in Example 2;
FIG. 5 shows the change in cell morphology after co-culture of GMOC and nucleus pulposus cells in Example 3;
FIG. 6 shows the results of regulation of cell phenotype by GMOC in Example 3;
FIG. 7 shows the results of enrichment of Mfge8 protein by GMOC in Example 3;
FIG. 8 shows the results of magnetic resonance imaging in Example 4;
FIG. 9 shows the results of X-ray imaging in Example 4.

### Detailed Description of Embodiments

In order to make the objectives, technical solutions and advantages of examples of the present disclosure clearer, the technical solutions in the examples of the present disclosure will be clearly and completely described below. If specific conditions are not specified in the examples, conventional conditions or conditions recommended by a manufacturer are followed. The reagents or instruments used therein for which manufacturers are not specified are all conventional products that are commercially available.

Unless otherwise specified, the terms "konjac glucomannan" and "konjac polysaccharide" of the present disclosure are used interchangeably, both referring to a water-soluble macromolecular polysaccharide, which is a polymer of glucose and mannan linked by β-1,4-glycosidic bonds.

Unless otherwise specified, the terms "nanoparticle" and "glucomannan caprylate nanoparticle" of the present disclosure are used interchangeably, both referring to a substance obtained by subjecting the glucomannan caprylate to a nanonisation treatment by methods known in the art. The methods and conditions of the nanonisation treatment are not limited to those listed in the present disclosure, and similar treatments may also be carried out with reference to the relevant prior art.

Unless otherwise specified, the degree of substitution of the present disclosure refers to the number of hydroxyl groups at the 2, 3, and 6 positions of the saccharide ring that are substituted with corresponding groups during the esterification reaction of the konjac glucomannan. It can be understood that when the hydroxyl groups at the 2, 3, and 6 positions of the saccharide ring are completely substituted with corresponding groups, the degree of substitution is 3.

The degree of substitution can be calculated by using proton nuclear magnetic resonance spectroscopy, with the specific formula as follows: (integrated area of the methyl proton peak of the ester group in the reaction product/3)/(integrated area of the proton peaks on the saccharide ring/7).

The use of the konjac glucomannan and the derivative thereof, the glucomannan caprylate (GMOC), the nanoparticles and the coating obtained from the glucomannan caprylate, the preparation method for the nanoparticles, and the use of the glucomannan caprylate, the nanoparticles or the coating provided in the present disclosure are described in detail below.

The present disclosure provides the use of a konjac glucomannan and a derivative thereof, a glucomannan caprylate, nanoparticles and a coating obtained from the glucomannan caprylate, a preparation method for the nanoparticles, and the use of the glucomannan caprylate, the nanoparticles or the coating.

The present disclosure proposes the use of a konjac glucomannan (KGM) and a derivative thereof as extracellular matrix fillers.

The diverse and flexible molecular conformations of polysaccharides endow them with tremendous potential for tissue repair. The konjac glucomannan, as a water-soluble macromolecular polysaccharide, is a polymer of glucose and mannan linked by beta-1,4-glycosidic bonds in konjac tubers. In addition, the polysaccharide molecule can be modified to introduce an additional functional group to exert specific biological effects. For example, multiple acetyl groups are modified onto the polysaccharide by means of an esterification reaction to regulate cellular functions.

At the same time, the konjac glucomannan has excellent anti-enzymatic hydrolysis properties, enabling it to remain locally and stably for a long time, thereby supplementing the ECM lost due to degeneration, and ultimately achieving the therapeutic purpose of delaying intervertebral disc degeneration by improving the microenvironment of the degenerated intervertebral disc tissue.

The present disclosure prepares a filler material by selecting a konjac polysaccharide (KGM), which is not composed of nucleus pulposus ECM, and a derivative thereof, which can effectively resist degradation by matrix degrading enzymes common in situ, thereby achieving long-term retention of the filler material at the defect site.

In an optional embodiment, the derivative of konjac glucomannan is a modified konjac glucomannan.

In an optional embodiment, the modification is to modify a polysaccharide molecule of the konjac glucomannan to introduce an additional functional group.

The functional group may be an acetyl group or an octanoyl group.

In an optional embodiment, the derivative of konjac glucomannan is a glucomannan caprylate.

In an optional embodiment, the degree of substitution of the glucomannan caprylate is 1.0-2.5. For example, the degree of substitution of the glucomannan caprylate is 1.0, 1.1, 1.2, 1.3, 1.4, 1.5, 1.6, 1.7, 1.8, 1.9, 2.0, 2.1, 2.2, 2.3, 2.4, or 2.4, or any range therebetween, e.g., 1.5-2.5, optionally 1.6-1.8. In a specific embodiment, the degree of substitution of the glucomannan caprylate is about 1.7, and may be, for example, 1.61, 1.62, 1.63, 1.64, 1.65, 1.66, 1.67, 1.68, 1.69, 1.70, 1.71, 1.72, 1.73, 1.74, 1.75, 1.76, 1.77, 1.78, 1.79, or 1.80, or any range between these values.

It is believed that without being bound by theory, the degree of substitution will have a certain influence on subsequent biological effects. If the degree of substitution is too low, the polarity of the product will change, and in the purification process of dissolving in chloroform and reprecipitating with alcohol, the product with a low degree of substitution will be removed from the chloroform solution in an insoluble form. At the same time, an excessively low degree of substitution also poses certain challenges to the preparation process during the subsequent preparation of the coating and nanoparticles. It is known in the art that the degree of substitution is controlled by fixing the reaction time and the equivalent of the reactants.

In an optional embodiment, the konjac glucomannan and the derivative thereof are in the form of nanoparticles or a coating.

The present disclosure also provides a glucomannan caprylate, which is obtained by modifying a konjac glucomannan by means of an esterification reaction.

In an optional embodiment, the degree of substitution of the glucomannan caprylate is 1.0-2.5. For example, the degree of substitution of the glucomannan caprylate is 1.0, 1.1, 1.2, 1.3, 1.4, 1.5, 1.6, 1.7, 1.8, 1.9, 2.0, 2.1, 2.2, 2.3, 2.4, or 2.4, or any range therebetween, e.g., 1.5-2.5, optionally 1.6-1.8. In a specific embodiment, the degree of substitution of the glucomannan caprylate is about 1.7, and may be, for example, 1.61, 1.62, 1.63, 1.64, 1.65, 1.66, 1.67, 1.68, 1.69, 1.70, 1.71, 1.72, 1.73, 1.74, 1.75, 1.76, 1.77, 1.78, 1.79, or 1.80, or any range between these values.

Accordingly, the method for synthesising the above glucomannan caprylate may comprise the step of: modifying a konjac glucomannan with an octanoyl group.

In some embodiments, the konjac glucomannan is reacted with trifluoroacetic anhydride (TFAA) and octanoic acid, the reaction solution obtained from the reaction is subjected to solid-liquid separation, the liquid phase is collected for first alcohol precipitation, a first precipitate obtained from the first alcohol precipitation is dissolved in chloroform to remove a product insoluble in chloroform, subsequently the liquid phase dissolved in chloroform is subjected to second alcohol precipitation, and a second precipitate obtained is collected, wherein the second precipitate can be used as a glucomannan caprylate.

The reaction equation involved in the above process is as follows:

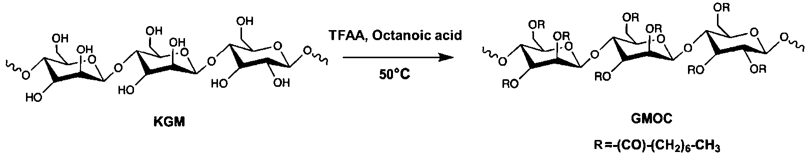

The above trifluoroacetic anhydride mainly serves as a catalyst in the synthesis reaction process, enabling the efficient production of the target product. The use of octanoic acid may ensure the simplicity, stability, and feasibility of the reaction, while avoiding the generation of by-products.

It should be noted that the product obtained from the first alcohol precipitation is glucomannan caprylate, which contains more impurities, and that a relatively pure product can be obtained by further purifying this product.

In some embodiments, the konjac glucomannan may be added to a first reaction solution obtained after a reaction of trifluoroacetic anhydride with octanoic acid for further reaction to obtain a second reaction solution; the second reaction solution is subjected to solid-liquid separation, the liquid phase is collected for first alcohol precipitation, a first precipitate obtained from the first alcohol precipitation is dissolved in chloroform to remove a product insoluble in chloroform, subsequently the liquid phase dissolved in chloroform is subjected to second alcohol precipitation, and a second precipitate obtained is collected.

For reference, the reaction of the trifluoroacetic anhydride with the octanoic acid may be carried out at 50°C-55°C, e.g., 50°C, 51°C, 52°C, 53°C, 54°C, or 55°C, for 15-25 min, e.g., 15 min , 18 min, 20 min, 22 min, or 25 min. For example, the range of the reaction temperature may be 51°C-54°C, 52°C-53°C, 51°C-53°C, or any value within the defined range. For example, the reaction time may be 18-22 min, 20-22 min, 15-22 min, or any value within the defined range. In some embodiments, the reaction of the trifluoroacetic anhydride with the octanoic acid is carried out at 50°C for 20 min.

The reaction of the konjac glucomannan with the first reaction solution may be carried out at 50°C-55°C (e.g., 50°C, 51°C , 52°C, 53°C, 54°C, or 55°C) for 3-4 h (e.g., 3 h, 3.2 h, 3.5 h, 3.8 h, or 4 h). For example, the range of the reaction temperature may be 51°C-54°C, 52°C-53°C, 51°C-53°C, or any value within the defined range. For example, the reaction time may be 3-3.8 h, 3.2-3.5 h, 3.2-3.7 h, or any value within the defined range. In some embodiments, the reaction of the konjac glucomannan with the first reaction solution is carried out at 50°C for 3.5 h.

The amount ratio of the konjac glucomannan, the trifluoroacetic anhydride, and the octanoic acid may be 500 mg : (8-12) mL : (12-14) mL, e.g., 500 mg : 8 mL : 12 mL, 500 mg : 8 mL : 13 mL, 500 mg : 8 mL : 14 mL, 500 mg : 10 mL : 12 mL, 500 mg : 10 mL : 13 mL, 500 mg : 10 mL : 14 mL, 500 mg : 12 mL : 12 mL, 500 mg : 12 mL : 13 mL, 500 mg : 12 mL : 14 mL. For example, the amount ratio of the konjac glucomannan, the trifluoroacetic anhydride, and the octanoic acid may be 500 mg : (10-12) mL : (13-14) mL or 500 mg : (9-11) mL : (12-14) mL, etc., or any value within the defined range. In some embodiments, the amount ratio of the konjac glucomannan, the trifluoroacetic anhydride, and the octanoic acid is 500 mg : 10 mL : 13 mL.

The alcohol used in each of the above alcohol precipitation processes may be, exemplarily but not limitingly, ethanol, and the amount of ethanol may be 3-5 times the volume of the solution to be treated.

Further, the second precipitate collected may also be dried in vacuum.

It should be noted that, in other embodiments, the above reaction conditions may also be appropriately adjusted as needed, and in addition, the second precipitate may also be further dissolved in chloroform and the liquid phase dissolved in chloroform is subjected to third alcohol precipitation. In other words, the number of alcohol precipitations may be adjusted as needed.

Based on the above proposed glucomannan caprylate, the present disclosure also provides a nanoparticle, which is obtained by subjecting the above glucomannan caprylate to a nanonisation treatment.

For reference, the method for preparing the nanoparticle may comprise the step of: subjecting the glucomannan caprylate to a nanonisation treatment.

In some embodiments, the glucomannan caprylate may be subjected to an ultrasonic nanonisation treatment in the presence of polyvinyl alcohol (PVA), an organic solvent is further removed after the nanoparticles are collected, and drying is carried out.

In some embodiments, the glucomannan caprylate may be first dissolved in chloroform to obtain a glucomannan caprylate solution, and then the glucomannan caprylate solution is added dropwise to a polyvinyl alcohol solution at a concentration of 4%-6% at a volume ratio of 1 : 8-12, followed by ultrasonication at 15-20 Hz for 2-4 minutes. Excess PVA is added and stirred overnight, the organic solvent is removed by rotary evaporation, and finally, nanoparticles are obtained by freeze-drying.

The above-PVA can utilise its surfactant property to promote a more uniform dispersion of the nanoparticles generated by ultrasonication in the solution and maintain stability, thereby preventing the fusion or rupture of the nanoparticles.

It should be noted that the methods and conditions for the nanonisation treatment are not limited to those listed above, and similar treatments may also be carried out with reference to the relevant prior art.

Based on the above proposed glucomannan caprylate, the present disclosure also provides a glucomannan caprylate coating, which is obtained by coating with the glucomannan caprylate.

It should be noted that conventional methods can be used for the coating substrate and the coating method, which are not particularly limited herein.

In addition, the above glucomannan caprylate may also be prepared in different dosage forms as required, e.g., capsules, soft capsules, powders, granules, tablets, pills, electuaries, oral liquids, tinctures, or injections.

Further, the present disclosure also provides the use of the above glucomannan caprylate or the above glucomannan caprylate nanoparticle or the above glucomannan caprylate coating in the preparation of a drug or material for regulating functions of nucleus pulposus cells.

In some embodiments, the above glucomannan caprylate or the above nanoparticle or the above glucomannan caprylate coating can be used for preparing a drug or material for regulating the adhesion and physiological functions of nucleus pulposus cells.

In some embodiments, the above glucomannan caprylate or the above nanoparticle or the above glucomannan caprylate coating can be used for preparing a drug or material for protecting the phenotype of nucleus pulposus cells by interfering with cell-matrix and cell-cell interactions.

In some embodiments, the above glucomannan caprylate or the above nanoparticle or the above glucomannan caprylate coating can be used for preparing a drug or material for highly expressing a nucleus pulposus phenotype.

In some embodiments, the above glucomannan caprylate or the above nanoparticle or the above glucomannan caprylate coating can be used for preparing a drug or material for enriching milk fat globule epidermal growth factor 8 (Mfge8 protein) secreted by nucleus pulposus cells; wherein the nucleus pulposus phenotype may comprise, exemplarily but not limitingly, Collagen2, etc.

In addition, the present disclosure provides the use of the above glucomannan caprylate or the above glucomannan caprylate nanoparticle or the above glucomannan caprylate coating in the preparation of a drug or material for relieving or treating intervertebral disc degeneration.

In some embodiments, the above glucomannan caprylate or the above nanoparticle or the above glucomannan caprylate coating can be used for preparing a drug for delaying water loss of a nucleus pulposus tissue. In other embodiments, the above glucomannan caprylate or the above nanoparticle or the above glucomannan caprylate coating can be used for preparing a drug for alleviating a decrease in intervertebral disc space height.

Accordingly, the present disclosure also provides a drug, wherein an active ingredient of the drug comprises the above glucomannan caprylate or the above nanoparticle or the above glucomannan caprylate coating.

Similarly, the present disclosure also provides a repair material, wherein an active component of the repair material comprises the above glucomannan caprylate or the above nanoparticle or the above glucomannan caprylate coating.

Accordingly, the present disclosure provides a method for regulating functions of nucleus pulposus cells, comprising administering to a subject in need thereof or applying to a subject in need thereof a therapeutically effective amount of the glucomannan caprylate according to the above embodiment or the nanoparticle according to the above embodiment or the glucomannan caprylate coating according to the above embodiment.

In some embodiments, the administering or applying is carried out in the form of a drug or material, respectively.

In some embodiments, the regulating functions of nucleus pulposus cells is selected from one or more of:
regulating the adhesion and physiological functions of nucleus pulposus cells;
protecting the phenotype of nucleus pulposus cells by interfering with cell-matrix and cell-cell interactions;
highly expressing a nucleus pulposus phenotype; optionally, the nucleus pulposus phenotype comprises Collagen2;
enriching milk fat globule epidermal growth factor 8 secreted by nucleus pulposus cells; wherein the nucleus pulposus phenotype comprises Collagen2.

In some embodiment, the material is a repair material.

Accordingly, the present disclosure provides a method for relieving or treating intervertebral disc degeneration, comprising administering to a subject in need thereof or applying to a subject in need thereof a therapeutically effective amount of the glucomannan caprylate according to the above embodiment or the nanoparticle according to the above embodiment or the glucomannan caprylate coating according to the above embodiment.

In some embodiments, the administering or applying is carried out in the form of a drug or material, respectively.

In some embodiments, the relieving or treating intervertebral disc degeneration is selected from delaying a decrease in water of a nucleus pulposus tissue and/or alleviating a decrease in intervertebral disc space height.

In some embodiment, the material is a repair material.

Accordingly, the present disclosure provides the glucomannan caprylate according to the above embodiment or the nanoparticle according to above embodiment or the glucomannan caprylate coating according to above embodiment, for use in regulating functions of nucleus pulposus cells.

In some embodiments, the administering or applying is carried out in the form of a drug or material, respectively.

In some embodiments, the regulating functions of nucleus pulposus cells is selected from one or more of:
regulating the adhesion and physiological functions of nucleus pulposus cells;
protecting the phenotype of nucleus pulposus cells by interfering with cell-matrix and cell-cell interactions;
highly expressing a nucleus pulposus phenotype; optionally, the nucleus pulposus phenotype comprises Collagen2;
enriching milk fat globule epidermal growth factor 8 secreted by nucleus pulposus cells; wherein the nucleus pulposus phenotype comprises Collagen2.

In some embodiment, the material is a repair material.

Accordingly, the present disclosure provides the glucomannan caprylate according to the above embodiment or the nanoparticle according to the above embodiment or the glucomannan caprylate coating according to the above embodiment, for use in relieving or treating intervertebral disc degeneration.

In some embodiments, the administering or applying is carried out in the form of a drug or material, respectively.

In some embodiments, the relieving or treating intervertebral disc degeneration is selected from delaying a decrease in water of a nucleus pulposus tissue and/or alleviating a decrease in intervertebral disc space height.

In some embodiment, the material is a repair material.

It should be noted that in the prior art, supplementing the intervertebral disc with exogenous collagen or hyaluronic acid can increase tissue water content and improve tissue microenvironment in a short period of time. However, abundantly expressed matrix degrading enzymes may further degrade the supplemented ECM, leading to the possibility of repair failure.

In cell-cell and cell-ECM interactions, Mfge8, as a "bridging protein" is secreted by cells and expressed in cell membranes and ECM, and is involved in mediating cell recognition and adhesion behaviours, thereby further regulating physiological functions. In the present disclosure, by applying an esterification reaction to introduce multiple acetyl groups onto the polysaccharide, the modified product exhibits an extremely high ability to enrich Mfge8 protein. By preparing polysaccharide coatings and nanoparticles and bringing them into contact with nucleus pulposus cells in vitro and in vivo, respectively, the functional changes of nucleus pulposus cells are mediated through the enrichment of Mfge8 protein. At the same time, the polysaccharide material has excellent anti-enzymatic hydrolysis properties, enabling it to remain locally and stably for a long time, thereby supplementing the ECM lost due to degeneration, and ultimately achieving the therapeutic purpose of delaying intervertebral disc degeneration by improving the microenvironment of the degenerated intervertebral disc tissue.

The konjac glucomannan and the derivative thereof provided by the present disclosure can be used as extracellular matrix fillers, and glucomannan caprylate obtained by modifying the konjac glucomannan by means of an esterification reaction, and nanoparticles or a glucomannan caprylate coating further obtained from the glucomannan caprylate can both be used for preparing drugs or materials for regulating functions of nucleus pulposus cells, and in particular, can be used for preparing drugs or materials for relieving or treating intervertebral disc degeneration. The glucomannan caprylate has the characteristics of being non-toxic, easily available and highly histocompatible, and can be prepared into different dosage forms according to requirements. The present disclosure provides a novel idea to improve the microenvironment of intervertebral disc degeneration, and also provides a novel use of a biomaterial based on a polysaccharide derivative.

The characteristics and performance of the present disclosure will be further described in detail below in combination with the examples.

### Example 1

### Verification of the anti-enzymatic hydrolysis property of KGM

50 mg of KGM and 50 mg of hyaluronic acid (molecular weight: 180 K) were each added to 10 mL of a buffer solution (PBS salt solution, pH = 5.3). After stirring for complete dissolution, 2 mg of hyaluronidase was added to each, the reaction was carried out fully at 37°C for 6 h, the temperature was then raised to 90°C to inactivate the enzyme for 5 min, and the reaction was terminated. After cooling, the sample was filtered through a 0.22 µm filter membrane to remove insoluble particles. Mass spectrometry was used to detect the presence of oligosaccharide products in the reaction solution after enzymatic hydrolysis to verify the anti-enzymatic hydrolysis property of the substrate.

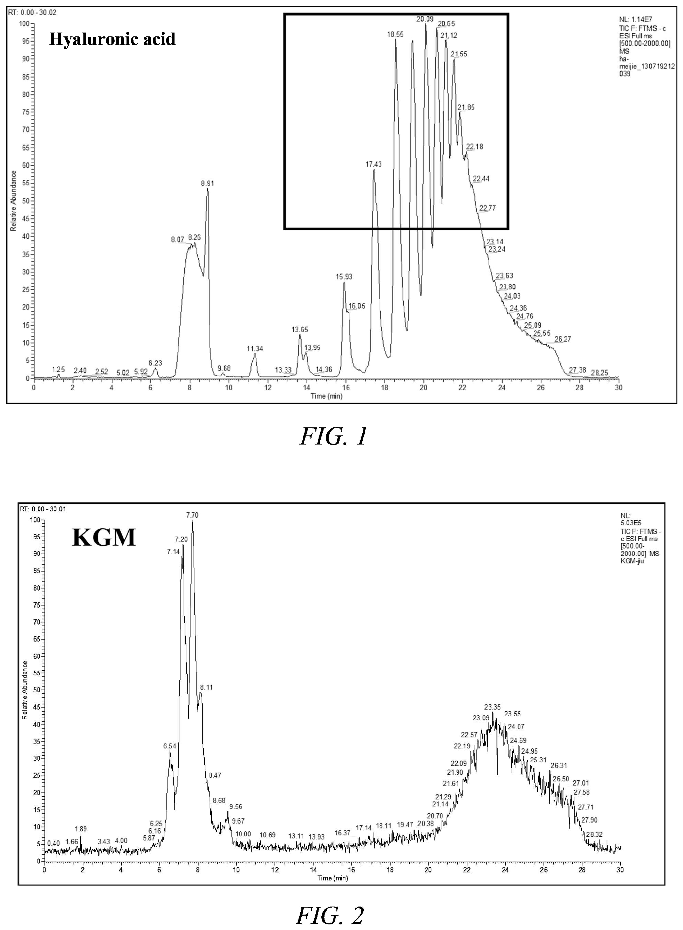

The mass spectrometry results are shown in FIG. 1 and FIG. 2, where in FIG.1, with hyaluronic acid as a control group, a large number of oligosaccharide peaks appeared after enzymatic hydrolysis, indicating that the hyaluronic acid was enzymatically hydrolysed; while in FIG. 2, KGM showed no significant oligosaccharide peaks, indicating that KGM possesses good anti-enzymatic hydrolysis property.

### Example 2

### Synthesis of GMOC by esterification reaction

13.2 mL of octanoic acid was weighed and slowly added dropwise to 10 mL of TFAA, and reacted in an oil bath at 50°C for 20 min to obtain a first reaction solution. 500 mg of KGM was added to the first reaction solution and reacted for 3.5 h to obtain a second reaction solution. The second reaction solution was centrifuged, and the supernatant was added dropwise to four volumes of ethanol solution for first alcohol precipitation. After centrifugation, a first precipitate was collected and further dissolved in chloroform to remove insoluble products. Subsequently, the liquid phase dissolved in chloroform was subjected to second alcohol precipitation, and the obtained second precipitate, i.e., the modified reaction product GMOC, was collected, and dried in vacuum for later use.

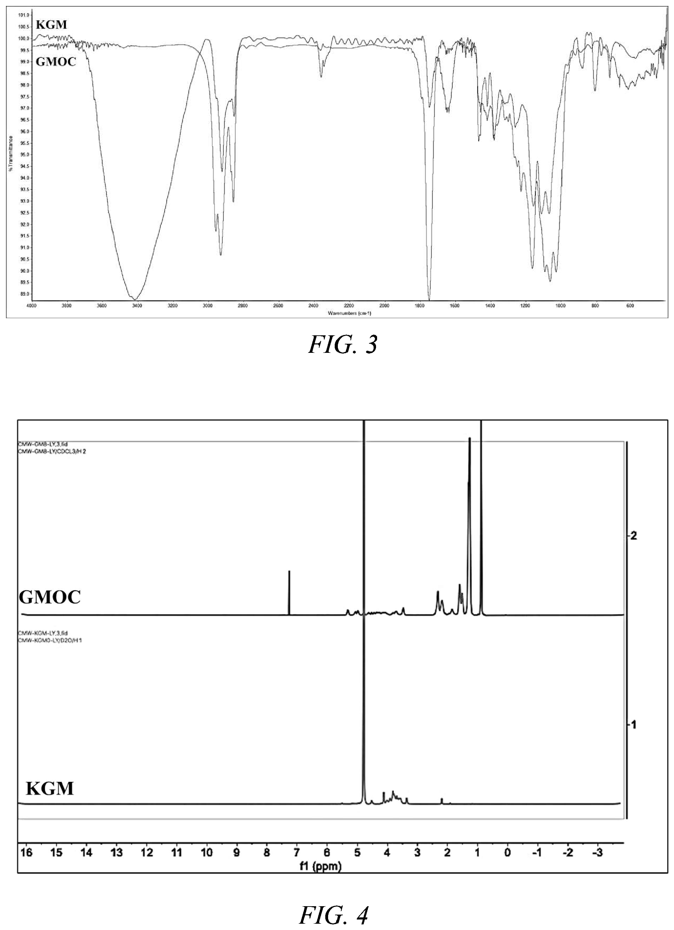

1 mg of the obtained GMOC was characterised by infrared spectroscopy and nuclear magnetic resonance, and the successful modification was observed. As shown in FIG. 3 and FIG. 4, it was calculated that by controlling the reaction time, a GMOC product with a degree of substitution of about 1.77 could be stably obtained.

### Example 3

### Verification of the effect of GMOC in regulating functions of nucleus pulposus cells

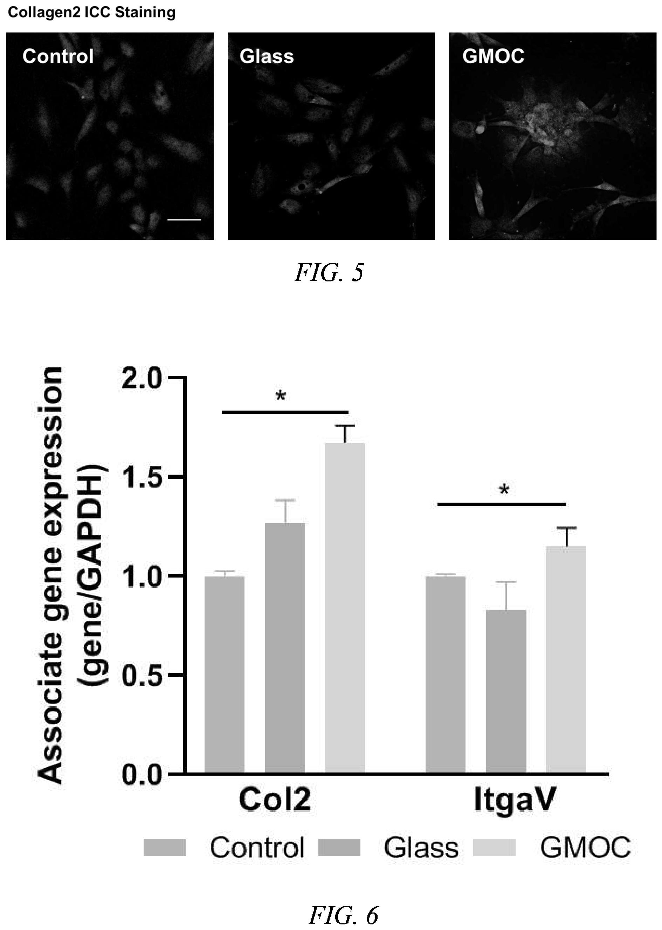

The GMOC prepared in Example 2 was coated on the surface of a cell coverslip and co-cultured with nucleus pulposus cells. The expression of the phenotype of nucleus pulposus cells and the changes in cell morphology were observed using cellular immunofluorescence staining. The results are shown in FIG. 5. It can be seen from the figure that: the nucleus pulposus cells exhibited significant colony-like growth on GMOC, and were able to highly express nucleus pulposus phenotypes such as Collagen2.

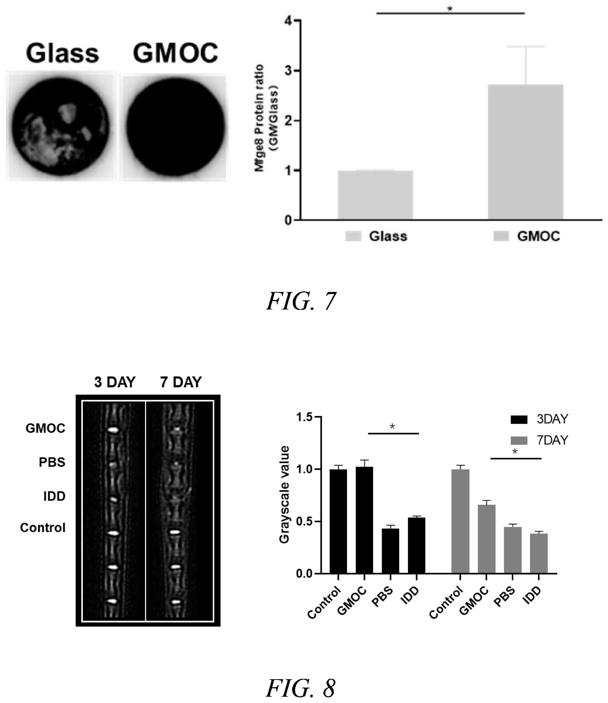

Further proteomic analysis suggested that the GMOC coating was able to significantly adsorb Mfge8 protein secreted by nucleus pulposus cells, which was confirmed in subsequent validation experiments (as shown in FIG. 7).

Herein, the proteomic analysis refers to the detection of the types of nucleus pulposus cell membrane proteins specifically adsorbed by GMOC using 4D-label free technology. The steps were as follows: after co-culturing the nucleus pulposus cell membrane with GMOC for 24 hours, the mixture was repeatedly washed three times with PBS, the proteins adsorbed on the GMOC surface were digested into peptides using trypsin, and the peptides were desalted, and then loaded onto the instrument for detection.

The experiment corresponding to the experiment of FIG. 7 was carried out as follows: nucleus pulposus cells were seeded on the surfaces of Glass and GMOC materials and cultured for 24 hours. EDTA was used to remove cells adhering to the surfaces. After washing three times with PBS, the Glass and GMOC materials were each incubated with a primary antibody against mfge8 overnight at 4°C, and washed three times with PBS. An HRP-labelled secondary antibody was added, incubated at room temperature for 1 hour, and washed three times with PBS. An ECL chemiluminescent solution was added, and exposure detection was carried out under an instrument to detect the adsorption of mfge8 protein on the surface of the material.

In FIG. 6 and FIG. 7, Control represents a conventionally used cell culture plate; Glass represents a cell coverslip, i.e., a glass slide; and GMOC represents the glucomannan coating provided by the present disclosure, which is coated on the surface of the cell coverslip.

### Example 4

### Verification of the therapeutic effect of GMOC in relieving intervertebral disc degeneration in an animal model

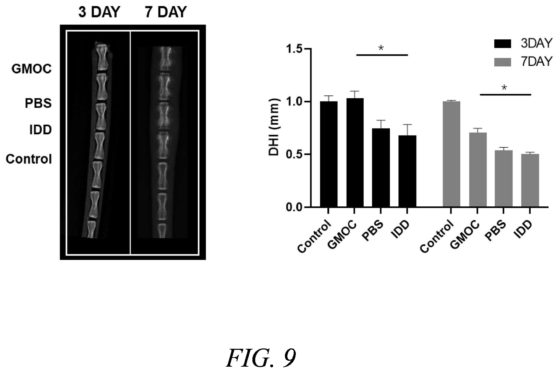

Female SD rats aged 8 weeks and weighing 250 g (obtained from the Animal Research Core, Faculty of Health Sciences, University of Macau) were anaesthetised, and the intervertebral disc space was punctured halfway using a 21G needle to establish an intervertebral disc degeneration (IDD) model. 10 µL of a 5% (w/v) GMOC solution in PBS was injected in situ into the defect site using a microsyringe as the treatment group, wherein GMOC was prepared according to Example 2. The defect segment injected with an equal amount of PBS served as a control group. 3.0 t magnetic resonance imaging and X-ray were used to detect the water content of the nucleus pulposus tissue and the intervertebral disc space height at 3 and 7 days after treatment, respectively, to evaluate the therapeutic effect of GMOC. The results are shown in FIG. 8 and FIG. 9.

Herein, control represents a healthy control group, i.e., a control group without any treatment; the IDD group represents a degeneration group, i.e., puncturing only, without any treatment; the PBS group represents injection with a PBS solution after puncture; and the GMOC group represents injection with a GMOC solution after puncture.

It can be seen from FIG. 8 and FIG. 9 that, compared with the IDD group, the GMOC treatment was able to significantly delay the water loss of the nucleus pulposus tissue and alleviate the extent of the decrease in intervertebral disc space height.

FIG. 8 shows the water content of the nucleus pulposus tissue as evaluated on MR T2 images. By calculating the grayscale value of the nucleus pulposus tissue, changes in the water content of the nucleus pulposus tissue can be reflected. Compared with the PBS and IDD groups, the tissue water content was still maintained on day 7 after GMOC treatment, which was reflected in less loss of tissue signal.

FIG. 9 shows the degree of intervertebral disc tissue degeneration, which was reflected by measuring the height between adjacent intervertebral disc spaces (DHI) using X-RAY. Compared with the PBS and IDD groups, a certain intervertebral disc space height was still maintained on day 7 after GMOC treatment, indicating that sufficient nucleus pulposus tissue remained therein as support.

In summary, the present disclosure provides a glycomimetic biomaterial, i.e., a glucomannan caprylate (GMOC), which is obtained by modifying a konjac polysaccharide (KGM) by means of an esterification reaction. GMOC can regulate the adhesion and physiological functions of nucleus pulposus cells by enriching milk fat globule epidermal growth factor 8 (Mfge8), and further protect the phenotype of nucleus pulposus cells by interfering with cell-matrix and cell-cell interactions, thereby improving the microenvironment of intervertebral disc degeneration. The saccharide derivative material (GMOC) has the characteristics of being non-toxic, easily available and highly histocompatible, and can be prepared into different dosage forms according to requirements. On this basis, the present disclosure provides a novel idea to improve the microenvironment of intervertebral disc degeneration, and a novel use of a biomaterial based on the polysaccharide derivative.

The above is only preferred examples of the present disclosure and is not intended to limit the present disclosure. For a person skilled in the art, the present disclosure may have various changes and variations. Any modifications, equivalent substitutions, improvements, etc., made within the spirit and principle of the present disclosure shall fall within the scope of protection of the present disclosure.

### Industrial Applicability

The present disclosure provides the use of a konjac glucomannan and a derivative thereof. The konjac glucomannan and the derivative thereof can be used as extracellular matrix fillers, and glucomannan caprylate obtained by modifying the konjac glucomannan by means of an esterification reaction, and nanoparticles or a glucomannan caprylate coating further obtained from the glucomannan caprylate can both be used for preparing drugs or materials for regulating functions of nucleus pulposus cells, and in particular, can be used for preparing drugs or materials for relieving or treating intervertebral disc degeneration. The glucomannan caprylate has the characteristics of being non-toxic, easily available and highly histocompatible, can be prepared into different dosage forms according to requirements, and has excellent industrial applicability.

## Claims

1. Use of a konjac glucomannan and a derivative thereof as extracellular matrix fillers.

2. The use according to claim 1, **characterised in that** the derivative of konjac glucomannan is a modified konjac glucomannan;
optionally, the modification is to modify a polysaccharide molecule of the konjac glucomannan to introduce an additional functional group;
optionally, the functional group is an acetyl group or an octanoyl group;
optionally, the derivative of konjac glucomannan is a glucomannan caprylate;
optionally, the degree of substitution of the glucomannan caprylate is 1.0-2.5; optionally, the degree of substitution of the glucomannan caprylate is 1.5-2.5; optionally, the degree of substitution of the glucomannan caprylate is 1.6-1.8;
optionally, the konjac glucomannan and the derivative thereof are in the form of nanoparticles or a coating.

3. A glucomannan caprylate, **characterised in that** the glucomannan caprylate is obtained by modifying a konjac glucomannan by means of an esterification reaction;
optionally, the degree of substitution of the glucomannan caprylate is 1.0-2.5; optionally, the degree of substitution of the glucomannan caprylate is 1.5-2.5; optionally, the degree of substitution of the glucomannan caprylate is 1.6-1.8.

4. A method for synthesising the glucomannan caprylate according to claim 3, **characterised by** comprising the step of: modifying a konjac glucomannan with an octanoyl group;
optionally, the konjac glucomannan is reacted with trifluoroacetic anhydride and octanoic acid, the reaction solution obtained from the reaction is subjected to solid-liquid separation, the liquid phase is collected for first alcohol precipitation, a first precipitate obtained from the first alcohol precipitation is dissolved in chloroform to remove a product insoluble in chloroform, subsequently the liquid phase dissolved in chloroform is subjected to second alcohol precipitation, and a second precipitate obtained is collected;
optionally, the konjac glucomannan is added to a first reaction solution obtained after a reaction of trifluoroacetic anhydride with octanoic acid for further reaction to obtain a second reaction solution; the second reaction solution is subjected to solid-liquid separation, the liquid phase is collected for first alcohol precipitation, a first precipitate obtained from the first alcohol precipitation is dissolved in chloroform to remove a product insoluble in chloroform, subsequently the liquid phase dissolved in chloroform is subjected to second alcohol precipitation, and a second precipitate obtained is collected;
optionally, the reaction of the trifluoroacetic anhydride with the octanoic acid is carried out at 50°C-55°C for 15-25 min;
optionally, the reaction of the konjac glucomannan with the first reaction solution is carried out at 50°C-55°C for 3-4 h;
optionally, the amount ratio of the konjac glucomannan, the trifluoroacetic anhydride, and the octanoic acid is 500 mg : (8-12) mL : (12-14) mL.

5. A nanoparticle, **characterised in that** the nanoparticle is obtained by subjecting the glucomannan caprylate according to claim 3 to a nanonisation treatment.

6. A method for preparing the nanoparticle according to claim 5, **characterised by** comprising the step of: subjecting the glucomannan caprylate to a nanonisation treatment;
optionally, the glucomannan caprylate is subjected to an ultrasonic nanonisation treatment in the presence of polyvinyl alcohol, an organic solvent is removed, and drying is carried out.

7. A glucomannan caprylate coating, **characterised in that** the glucomannan caprylate is obtained by coating with the glucomannan caprylate according to claim 3.

8. Use of the glucomannan caprylate according to claim 3 or the nanoparticle according to claim 5 or the glucomannan caprylate coating according to claim 7 in the preparation of a drug or material for regulating functions of nucleus pulposus cells;
optionally, the glucomannan caprylate or the nanoparticle or the glucomannan caprylate coating is used for preparing a drug or material for regulating the adhesion and physiological functions of nucleus pulposus cells;
optionally, the glucomannan caprylate or the nanoparticle or the glucomannan caprylate coating is used for preparing a drug or material for protecting the phenotype of nucleus pulposus cells by interfering with cell-matrix and cell-cell interactions;
optionally, the glucomannan caprylate or the nanoparticle or the glucomannan caprylate coating is used for preparing a drug or material for highly expressing a nucleus pulposus phenotype; optionally, the nucleus pulposus phenotype comprises Collagen2;
optionally, the glucomannan caprylate or the nanoparticle or the glucomannan caprylate coating is used for preparing a drug or material for enriching milk fat globule epidermal growth factor 8 secreted by nucleus pulposus cells; wherein the nucleus pulposus phenotype comprises Collagen2;
optionally, the material is a repair material.

9. Use of the glucomannan caprylate according to claim 3 or the nanoparticle according to claim 5 or the glucomannan caprylate coating according to claim 7 in the preparation of a drug or material for relieving or treating intervertebral disc degeneration;
optionally, the glucomannan caprylate or the nanoparticle or the glucomannan caprylate coating is used for preparing a drug for delaying water loss of a nucleus pulposus tissue, or, for preparing a drug for alleviating a decrease in intervertebral disc space height;
optionally, the material is a repair material.

10. A drug, **characterised in that** an active ingredient of the drug comprises the glucomannan caprylate according to claim 3 or comprises the nanoparticle according to claim 5 or the glucomannan caprylate coating according to claim 7.

11. A repair material, **characterised in that** an active component of the repair material comprises the glucomannan caprylate according to claim 3 or comprises the nanoparticle according to claim 5 or the glucomannan caprylate coating according to claim 7.

12. A method for regulating functions of nucleus pulposus cells, **characterised by** comprising administering to a subject in need thereof or applying to a subject in need thereof a therapeutically effective amount of the glucomannan caprylate according to claim 3 or the nanoparticle according to claim 5 or the glucomannan caprylate coating according to claim 7;
optionally, the administering or applying is carried out in the form of a drug or material, respectively;
optionally, the regulating functions of nucleus pulposus cells is selected from one or more of:
regulating the adhesion and physiological functions of nucleus pulposus cells;
protecting the phenotype of nucleus pulposus cells by interfering with cell-matrix and cell-cell interactions;
highly expressing a nucleus pulposus phenotype; optionally, the nucleus pulposus phenotype comprises Collagen2;
enriching milk fat globule epidermal growth factor 8 secreted by nucleus pulposus cells; wherein the nucleus pulposus phenotype comprises Collagen2;
optionally, the material is a repair material.

13. A method for relieving or treating intervertebral disc degeneration, **characterised by** comprising administering to a subject in need thereof or applying to a subject in need thereof a therapeutically effective amount of the glucomannan caprylate according to claim 3 or the nanoparticle according to claim 5 or the glucomannan caprylate coating according to claim 7;
optionally, the administering or applying is carried out in the form of a drug or material, respectively;
optionally, the relieving or treating intervertebral disc degeneration is selected from delaying water of a nucleus pulposus tissue and/or alleviating a decrease in intervertebral disc space height;
optionally, the material is a repair material.

14. The glucomannan caprylate according to claim 3 or the nanoparticle according to claim 5 or the glucomannan caprylate coating according to claim 7, for use in regulating functions of nucleus pulposus cells;
optionally, the administering or applying is carried out in the form of a drug or material, respectively;
optionally, the regulating functions of nucleus pulposus cells is selected from one or more of:
regulating the adhesion and physiological functions of nucleus pulposus cells;
protecting the phenotype of nucleus pulposus cells by interfering with cell-matrix and cell-cell interactions;
highly expressing a nucleus pulposus phenotype; optionally, the nucleus pulposus phenotype comprises Collagen2;
enriching milk fat globule epidermal growth factor 8 secreted by nucleus pulposus cells; wherein the nucleus pulposus phenotype comprises Collagen2;
optionally, the material is a repair material.

15. The glucomannan caprylate according to claim 3 or the nanoparticle according to claim 5 or the glucomannan caprylate coating according to claim 7, for use in relieving or treating intervertebral disc degeneration;
optionally, the administering or applying is carried out in the form of a drug or material, respectively;
optionally, the relieving or treating intervertebral disc degeneration is selected from delaying water of a nucleus pulposus tissue and/or alleviating a decrease in intervertebral disc space height;
optionally, the material is a repair material.
